# EUROPEAN PATENT SPECIFICATION

(11) **EP 1 509 500 B1**
(45) Date of publication and mention of the grant of the patent: **30.11.2005**
(21) Application number: 03727362.0
(22) Date of filing: 25.04.2003
(51) Int. Cl.: C07D 207/34

(54) **PROCESS FOR THE PREPARATION OF THE AMORPHOUS FORM OF ATORVASTATIN CALCIUM SALT**
VERFAHREN ZUR HERSTELLUNG DER AMORPHEN FORM VON ATORVASTATIN CALZIUM SALZ
PROCEDE DE PREPARATION DE LA FORME AMORPHE D'UN SEL D'ATORVASTATINE CALCIQUE

(30) Priority: 29.04.2002 IT MI20020907
(43) Date of publication of application: 02.03.2005
(73) Proprietor: CHEMI S.p.A., 20092 Cinisello Balsamo (Milano) (IT)
(72) Inventor: TURCHETTA, Stefano, I-00133 Roma (IT); MASSARDO, Pietro, I-00154 Roma (IT); TUOZZI, Angela, I-00125 Roma (IT)
(74) Representative: Gervasi, Gemma
(86) International application number: PCT/EP2003/004313
(87) International publication number: WO 2003/093233

(56) References cited:
- WO-A-00/71116
- WO-A-01/28999
- WO-A-01/42209
- WO-A-02/059087
- WO-A-03/018547
- US-B1- 6 274 740

## Description

### FIELD OF THE INVENTION

The present invention relates to a reproducible method for preparing the amorphous form of atorvastatin calcium salt, in such a way as to be easily filtered, and with a purity superior to the initial crystalline form.

### STATE OF THE ART

Atorvastatin is a well known active pharmaceutical principle widely used for the treatment of diseases caused by hypercholesterolaemia. US4681893, US5273995, US6121461, US5969156 refer to the preparation of the product both in amorphous and crystalline form. While the production of a composition with a well defined crystalline form can in many cases be advantageous from the point of view of stability and from the point of view of the dosage of the active principle in the pharmaceutical formulation, in some cases this can give rise to water solubility and bioavailability differences. This is the case with atorvastatin where the corresponding amorphous form demonstrates superior characteristics of water solubility and bioavailability than the corresponding crystalline form. On the other hand the known processes for producing atorvastatin in amorphous form present problems due to the poor reproducibility and/or poor workability of the product or are not suitable for scale up to industrial production.

For example in US6087511 and US6274740 are described the preparation of the amorphous form of atorvastatin calcium salt starting from the crystalline form (I) by evaporating the solution of the product in organic solvents such as tetrahydrofuran or tetrahydrofuran-toluene, until a foamy solid residue is obtained. This method presents considerable drawbacks from the point of view of industrial application. In regard to the workability of the product, at the end of the preparation a fragile foam is obtained which must be broken up in the reactor and must be discharged from the reactor as a solid.

W0007116 reports the production of atorvastatin in amorphous form from a solution of the product in a non-hydroxylic solvent. In this case high levels of hydrocarbon are necessary to obtain the desired product.

W00128999 describes the preparation of amorphous atorvastatin by precipitating the product from solutions of atorvastatin calcium salt in lower alkanols. In this case enormous quantities of alcohols are necessary to obtain the desired product.

### TECHNICAL PROBLEM

It was therefore considered necessary to provide a method for producing atorvastatin in amorphous form that was economically advantageous and at the same time industrially scalable.

### SUMMARY OF THE INVENTION

The applicant has unexpectedly found that atorvastatin calcium salt can be produced in amorphous form, by a method that does not present the inconveniences of the state of the art.

In particular the process of the present invention comprises:
a)dissolving the atorvastatin calcium salt in an organic solvent miscible with water,
b) gradually adding said solution to water while stirring,
c) filtering and vacuum drying the solid obtained.

### DESCRIPTION OF THE FIGURE

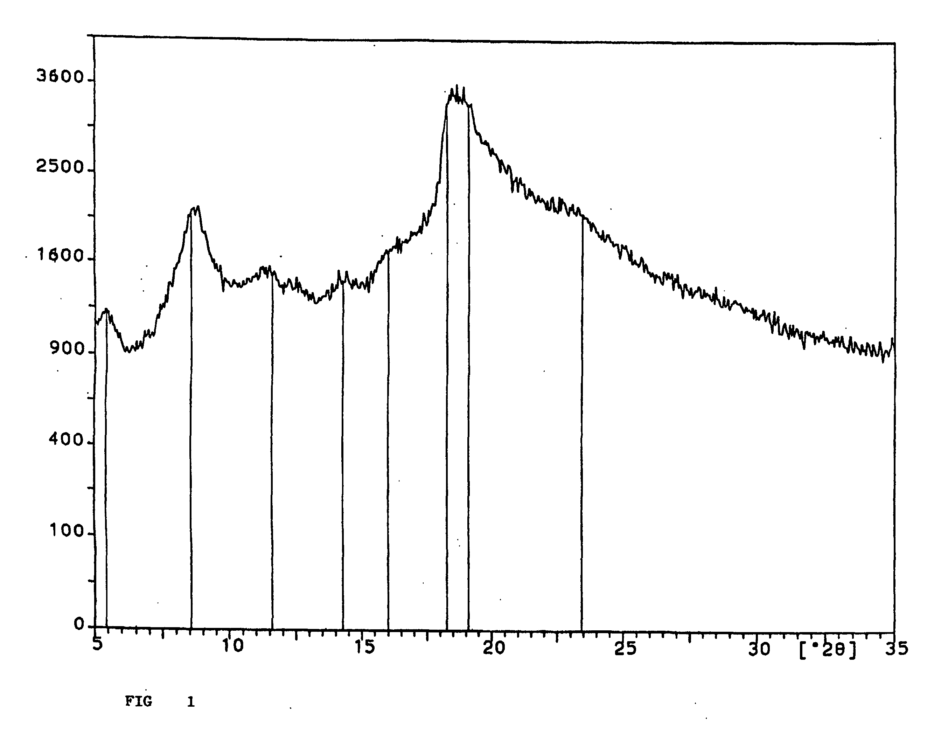

Figure 1 shows the x-ray diffraction spectrum of the amorphous atorvastatin prepared as described in example 1.

The measurements were made at the wavelengths Kα1 and Kα2 using 5.000° for angle 2θ and 35.000° for the final angle. In this figure,in ordinates the number of counts per second is reported, and in abscissae the values of the angle 2θ.

### DETAILED DESCRIPTION OF THE INVENTION

The atorvastatin used as starting material can be either crystalline or amorphous. Consequently the atorvastatin used in stage (a) of the process of the present invention can therefore be crystalline atorvastatin of form (I), (II) and (IV) as described in US5969156 form (III) as described in US6121461 or the amorphous form derived from the reaction described in US5273995.

Preferably this latter type of atorvastatin is used.

The water miscible solvent is preferably chosen from: tetrahydrofuran, dimethylsulphoxide, dimethylacetamide, dimethylformamide, N-methylpyrrolidone, sulfolane. The additional advantage of this method is that the product obtained, whose amorphous nature is confirmed by the relative x-ray diffraction spectrum, has a higher purity than the starting product.

Preferably the atorvastatin calcium salt is dissolved in a quantity of organic solvent between 0.5 and 20, more preferably between 1 and 10 and even more preferably between 1 and 5 ml/gram of the atorvastatin calcium salt in crystalline form. The amount of water, to which the atorvastatin in organic solvent is slowly added, is preferably between 5 and 100, more preferably between 10 and 50, and even more preferably between 10 and 30 ml/gram of atorvastatin calcium salt in crystalline form. The temperature of the constantly stirred water is between 5 and 40°C, preferably between 10 and 30°C. Preferably the water soluble organic solvent is tetrahydrofuran. As the solution of atorvastatin calcium salt in the organic solvent is dripped onto the stirred water, the formation of a solid is observed which becomes more consistent as the addition proceeds. At the end of the addition the mixture is stirred for a period of time between 0.5 and 5 hours, preferably between 1 .and 3 hours and even more preferably between 2 and 3 hours at a temperature of between 5 and 40°C and preferably between 10 and 30°C, after which the suspension is filtered and the solid washed with water.

A further advantage of this method lies in the good filterability of the solid obtained due to the addition of the organic solution to the water. Indeed the addition of water to the organic solution results in the formation of gummy masses which cannot be filtered or stirred.

Some illustrative examples are given hereinafter of the preparation process according to the present invention.

### EXAMPLE 1

5 g of crude amorphous atorvastatin calcium salt derived from the reaction mixture of the process described in US5273995 are dissolved in 15 ml of THF and loaded into a dropping funnel. The funnel is placed above a 250 ml reaction flask equipped with mechanical stirrer. 100 ml of deionized water are loaded into the reactor and maintained at 22-25°C and from the dropping funnel the THF solution is added to the water, resulting in the formation of a white solid. When the addition is complete the suspension is cooled to 10°C while stirring and maintained at that temperature for 1 hour. The precipitate is then filtered off under reduced pressure and washed with 20 ml of deionized water. 13.4 g of a wet product is obtained which, after drying for 12 hours at 40°C under reduced pressure (50mm Hg) gives rise to 4.8 g of atorvastatin calcium salt in amorphous form (yield 95%), of a purity superior to that of the initial crude atorvastatin evaluated by means of TLC as comparison.

Figure 1 shows the x-ray diffraction spectrum of the atorvastatin calcium salt in amorphous form thus obtained, a spectrum which is entirely in accordance with those already reported in the literature for such a product.

### EXAMPLE 2

A 500 ml reactor equipped with mechanical stirrer and dropping funnel is filled with 200 ml of deionized water, maintained at 22-25°C. 20g of crude amorphous atorvastatin calcium salt derived from the reaction mixture of the process described in US5273995 are dissolved in 30 ml of N,N-dimethylacetamide and loaded into the dropping funnel. The organic solution is then slowly dripped onto the water and a white solid is formed. At the end of the addition the mixture is stirred for about 1 hour at 22-25°C and is then cooled to 10°C and maintained at that temperature for 1 hour. The solid is filtered off, washed with 50 ml of cold deionized water and dried under vacuum at 40°C for 12 hours to give 18.2g of atorvastatin calcium salt in amorphous form (yield 91%) of a purity superior to the initial crude atorvastatin evaluated by means of TLC as comparison.

### EXAMPLE 3

The reaction is conducted starting from 20 g of atorvastatin calcium salt using the same conditions as in example 2, with the only difference that dimethylsulphoxide is used as the organic solvent miscible in water. After drying, 17.5 g of atorvastatin calcium salt in amorphous form are obtained (yield 87.5%) of a purity superior to the initial crude atorvastatin evaluated by means of TLC as comparison.

## Claims

1. Process for preparing atorvastatin calcium salt in amorphous form comprising
a) dissolving the atorvastatin calcium salt in an organic solvent miscible with water,
b) gradually adding said solution to water while stirring,
c) filtering and vacuum drying the solid obtained.

2. Process as claimed in claim 1, **characterised in that** atorvastatin used in stage (a) is amorphous atorvastatin.

3. Process as claimed in claim 1 or 2, **characterised in that** the water miscible solvent is chosen from tetrahydrofuran, dimethylsulphoxide, dimethylacetamide, dimethylformamide, N-methylpyrrolidone, sulfolane.

4. Process as claimed in claim 3 wherein said water miscible organic solvent is tetrahydrofuran..

5. Process as claimed in any one of claims 1-4, **characterised in that** in stage (a) atorvastatin calcium salt is dissolved in a quantity of water miscible organic solvent of between 0.5 and 20 ml/gram of atorvastatin calcium salt in crystalline form.

6. Process as claimed in claim 5, **characterised in that** said quantity of organic solvent is between 1 and 10 ml/gram of atorvastatin calcium salt in crystalline form.

7. Process as claimed in claim 6, **characterised in that** said quantity of organic solvent is between 1 and 5 ml/gram of atorvastatin calcium salt in crystalline form.

8. Process as claimed in any one of claims 1-7 **characterised in that** in stage (a) the quantity of water, to which the solution of atorvastatin in organic solvent is slowly added, is between 5 and 100 ml/gram of atorvastatin calcium salt in crystalline form.

9. Process as claimed in claim 8, **characterised in that** said quantity of water is between 10 and 50 ml/gram of atorvastatin calcium salt in crystalline form.

10. Process as claimed in claim 9, **characterised in that** said quantity of water is between 10 and 30 ml/gram of atorvastatin calcium salt in crystalline form

11. Process as claimed in any one of claims 1-10, **characterised in that** the water temperature in stage (a) is between 5 and 40°C.

12. Process as claimed in claim 11, **characterised in that** the said temperature is between 10 and 30°C.

13. Process as claimed in any one of claims 1-10 **characterised in that** when stage (a) has terminated, the mixture obtained is left under stirring at a temperature between 5 and 40°C for a period of time between 0.5 and 5 hours.

14. Process as claimed in claim 13, **characterised in that** when stage (a) has terminated, the mixture obtained is left under stirring at a temperature between 10 and 30°C for a time period between 1 and 3 hours.

## Patentansprüche

1. Verfahren zur Herstellung von Atorvastatin-Kalziumsalz in amorpher Form umfassend:
a) Auflösen von Atorvastatin-Kalziumsalz in einem mit Wasser mischbaren organischen Lösungsmittel,
b) unter Rühren allmähliche Zugabe genannter Lösung zu Wasser,
c) Filtern und Vakuumtrocknen des erhaltenen Feststoffes.

2. Verfahren gemäß Anspruch 1, **dadurch gekennzeichnet, dass** es sich bei Atorvastatin in Schritt (a) um amorphes Atorvastatin handelt.

3. Verfahren gemäß Anspruch 1 oder 2, **dadurch gekennzeichnet, dass** das mit Wasser mischbare Lösungsmittel ausgewählt wird unter Tetrahydrofuran, Dimethylsulphoxid, Dimethylacetamid, Dimethylformamid, N-methylpyrrolidon, Sulfolan.

4. Verfahren wie in Anspruch 3, wobei es sich bei dem mit Wasser mischbaren organischen Lösungsmittel um Tetrahydrofuran handelt.

5. Verfahren gemäß einem der Ansprüche 1-4, **dadurch gekennzeichnet, dass** in Schritt (a) Atorvastatin-Kalziumsalz mit Wasser mischbarem organischen Lösungsmittel in einer Menge von 0,5 bis 20 ml/Gramm Atorvastatin-Kalziumsalz in kristalliner Form aufgelöst wird.

6. Verfahren gemäß Anspruch 5, **dadurch gekennzeichnet, dass** die genannte Menge an organischem Lösungsmittel zwischen 1 und 10 ml/Gramm Atorvastatin-Kalziumsalz in kristalliner Form beträgt.

7. Verfahren gemäß Anspruch 6, **dadurch gekennzeichnet, dass** die genannte Menge an organischem Lösungsmittel zwischen 1 und 5 ml/Gramm Atorvastatin-Kalziumsalz in kristalliner Form beträgt.

8. Verfahren gemäß einem der Ansprüche 1-7, **dadurch gekennzeichnet, dass** in Schritt (a) die Menge an Wasser, zu der die Lösung aus Atorvastatin in organischem Lösungsmittel langsam hinzugegeben wird, zwischen 5 und 100 ml/Gramm Atorvastatin-Kalziumsalz in kristalliner Form beträgt.

9. Verfahren gemäß Anspruch 8, **dadurch gekennzeichnet, dass** die genannte Menge an Wasser, die zu Atorvastatin-Kalziumsalz in kristalliner Form zugegeben wird, zwischen 10 und 50 ml/Gramm beträgt.

10. Verfahren gemäß Anspruch 9, **dadurch gekennzeichnet, dass** die genannte Menge an Wasser, die zu Atorvastatin-Kalziumsalz in kristalliner Form zugegeben wird, zwischen 10 und 30 ml/Gramm beträgt.

11. Verfahren gemäß einem der Ansprüche 1-10, **dadurch gekennzeichnet, dass** die Wassertemperatur in Schritt (a) zwischen 5°C und 40°C liegt.

12. Verfahren gemäß Anspruch 11, **dadurch gekennzeichnet, dass** die genannte Temperatur zwischen 10°C und 30°C beträgt.

13. Verfahren gemäß einem der Ansprüche 1-10, **dadurch gekennzeichnet, dass** nach Beendigung von Schritt (a) die erhaltene Mischung bei einer Temperatur von 5°C bis 40°C über einen Zeitraum von 0,5 bis 5 Stunden gerührt wird.

14. Verfahren gemäß Anspruch 13, **dadurch gekennzeichnet, dass** nach Beendigung von Schritt (a) die erhaltene Mischung bei einer Temperatur von 10°C bis 30°C über einen Zeitraum von 1 bis 3 Stunden gerührt wird.

## Revendications

1. Procédé de préparation de sel d'atorvastatine calcique sous forme amorphe comprenant :
- (a) la dissolution du sel d'atorvastatine calcique dans un solvant organique miscible à l'eau,
- (b) l'addition progressive de ladite solution à l'eau, sous agitation,
- (c) la filtration et le séchage sous vide du solide obtenu.

2. Procédé selon la revendication 1, **caractérisé en ce que** l'atorvastatine utilisée à l'Etape (a) est de l'atorvastatine amorphe.

3. Procédé selon la revendication 1 ou la revendication 2, **caractérisé en ce que** le solvant miscible à l'eau est choisi parmi le tétrahydrofurane, le diméthylsulfoxyde, le diméthylacétamide, le diméthylformamide, la N-méthylpyrrolidone, le sulfolane.

4. Procédé selon la revendication 3, dans lequel ledit solvant organique miscible à l'eau est le tétrahydrofurane.

5. Procédé selon l'une quelconque des revendications 1 à 4, **caractérisé en ce qu'**à l'Etape (a), le sel d'atorvastatine calcique est dissous dans une quantité de solvant organique miscible à l'eau comprise entre 0,5 et 20 ml par gramme de sel d'atorvastatine calcique sous forme cristalline.

6. Procédé selon la revendication 5, **caractérisé en ce que** ladite quantité de solvant organique est comprise entre 1 et 10 ml par gramme de sel d'atorvastatine calcique sous forme cristalline.

7. Procédé selon la revendication 6, **caractérisé en ce que** ladite quantité de solvant organique est comprise entre 1 et 5 ml par gramme de sel d'atorvastatine calcique sous forme cristalline.

8. Procédé selon l'une quelconque des revendications 1 à 7, **caractérisé en ce qu'**à l'Etape (a), la quantité d'eau à laquelle la solution d'atorvastatine dans le solvant organique est ajoutée lentement, est comprise entre 5 et 100 ml par gramme de sel d'atorvastatine calcique sous forme cristalline.

9. Procédé selon la revendication 8, **caractérisé en ce que** ladite quantité d'eau est comprise entre 10 et 50 ml par gramme de sel d'atorvastatine calcique sous forme cristalline.

10. Procédé selon la revendication 9, **caractérisé en ce que** ladite quantité d'eau est comprise entre 10 et 30 ml par gramme de sel d'atorvastatine calcique sous forme cristalline.

11. Procédé selon l'une quelconque des revendications 1 à 10, **caractérisé en ce que** la température de l'eau à l'Etape (a) est comprise entre 5 et 40°C.

12. Procédé selon la revendication 11, **caractérisé en ce que** ladite température est comprise entre 10 et 30°C.

13. Procédé selon l'une quelconque des revendications 1 à 10, **caractérisé en ce qu'**une fois l'Etape (a) achevée, le mélange obtenu est laissé sous agitation à une température de 5 à 40°C pendant une période de temps compris entre 0,5 et 5 heures.

14. Procédé selon la revendication 13, **caractérisé en ce qu'**une fois l'Etape (a) achevée, le mélange obtenu est laissé sous agitation à une température de 10 à 30°C pendant une période de temps compris entre 1 et 3 heures.
